(19) Europäisches Patentamt
European Patent Office
Office européen des brevets

(11) Publication number: 0 035 328
A1

(12) # EUROPEAN PATENT APPLICATION

(21) Application number: 81300527.9

(22) Date of filing: 09.02.81

(51) Int. Cl.³: C 07 C 1/22, C 07 C 29/15, B 01 J 31/38

(30) Priority: 14.02.80 US 121447

(43) Date of publication of application: 09.09.81 Bulletin 81/36

(84) Designated Contracting States: BE DE FR GB IT NL

(71) Applicant: Ozyagcilar, Mehmet N.,
Apartment 807 3145 Queen Frederica Drive, Mississauga
Ontario L4Y3A7 (CA)

(72) Inventor: Ozyagcilar, Mehmet N.,
Apartment 807 3145 Queen Frederica Drive, Mississauga
Ontario L4Y3A7 (CA)

(74) Representative: Ritter, Stephen David et al, Mathys & Squire 10 Fleet Street, London EC4Y 1AY (GB)

(54) **Method of synthesizing hydrocarbons and alcohols.**

(57) Improved economies are achieved in the synthesis of hydrocarbons and alcohols from hydrogen/carbon monoxide mixtures by adding steam to a hydrogen-lean synthesis gas derived, e.g., from the gasification of a liquid or solid fossil fuel and carrying out the synthesis in the presence of a catalyst comprising hydrided titanium supported on a metal or intermetallic compound capable of forming an unstable hydride.

EP 0 035 328 A1

METHOD OF SYNTHESIZING HYDROCARBONS AND ALCOHOLS

This invention relates generally to the production of hydrocarbons and alcohols from mixtures of hydrogen and carbon monoxide, especially mixtures largely composed of hydrogen and carbon monoxide that are derived from the gasification of liquid and solid fuels, and more particularly, to the use of steam to improve the economy of such a process.

Background of the Invention

Prior art methods for the production of hydrocarbons such as methane and ethane have employed carbon monoxide and hydrogen over nickel catalysts of various types. The cost of nickel catalyst is quite high, both because of raw material costs and because of difficulties experienced in the manufacturing process, the latter requiring that the nickel be supported on some type of inert base. Moreover, the attrition of nickel catalyst is high because the processes that have been used require relatively high temperatures which in turn causes a sintering type of breakdown that rapidly decreases the activity of the catalyst as a function of time.

Nickel catalysts are also sensitive to poisoning by a number of impurities usually found in the hydrogen and carbon oxide reactants. For example, hydrogen sulfide has been found to poison nickel catalysts even in very small concentrations by forming nickel sulfide. The thermodynamics of the system is such that sulfur poisoning can be reversed by raising the temperature or increasing the hydrogen to hydrogen sulfide ratio in the feed or both. However, the use of higher operating temperatures with nickel catalysts is restricted by a sintering problem. Also the purity of the feed gas is inherently limited as a practical matter by the costs of purification.

The reaction of carbon monoxide with hydrogen in the presence of nickel catalyst requires temperatures in the range of 300° to 500°C. to produce significant reaction rates. Temperatures within this range cause relatively rapid deterioration of the nickel catalyst. In addition, very little ethane can be produced with nickel catalysts since the ethane reaction is favored only at lower temperatures. Furthermore, practically no alcohol formation is observed over nickel catalysts. In general, the formation of alcohols is believed to follow a mechanism different from that prevailing in the synthesis of hydrocarbons. Heretofore a different catalyst, namely, zinc oxide, was required for the synthesis of methanol.

A further problem restricting the use of prior art catalysts is carbonyl formation. The carbonyls of metals like nickel, ruthenium and iron are extremely toxic compounds. They also have very low boiling temperatures and thus would be present in a vapor phase at the temperatures required for the synthesis of methane, ethane and higher carbon compounds. Carbonyl formation causes depletion of the catalyst as well as posing a severe health and safety problem. Such problems with prior art catalysts can be avoided only by carefully controlling the operating temperature, pressure, carbon oxide to hydrogen ratio and other operation parameters and conditions.

Even at the elevated temperatures indicated, the reaction rates with nickel and other known catalysts are relatively slow and require a high residence time in the reactor vessel, which in turn results in a relatively slow production rate for the final product desired.

In my prior patent 4,139,551, a process is disclosed for producing methane and ethane from hydrogen and oxides of carbon that avoids a number of the foregoing disadvantages. In this prior process methane and ethane are produced from mixtures of hydrogen and one or more oxides of carbon by passing the mixture over an iron/titanium catalyst wherein the atomic ratio of titanium to iron is preferably about 1.1:1. By using the iron/titanium catalyst, lower temperatures and pressures can be used that are required with the nickel catalyst and the formation of toxic carbonyl compounds is avoided. Also higher reaction rates can be achieved.

While the process disclosed in U. S. patent 4,139,551 provides a number of important advantages over prior art processes using nickel catalysts, it still leaves something to be desired in cases where the starting material used is derived from the gasification of any of various fossil fuel sources, especially coal. Gaseous mixtures thus produced tend to contain carbon monoxide and hydrogen in proportions such that there is insufficient hydrogen to form the desired hydrocarbons. Thus, if such starting mixtures are used, additional hydrogen must be incorporated in the mixtures and such additional hydrogen is quite expensive.

## Objects of the Invention

It is a general object of the invention to provide a novel and improved process for the manufacture of hydrocarbons and alcohols from oxides of carbon and hydrogen. It is another object of the invention to manufacture hydrocarbons using less expensive raw materials. It is still another object of the invention to provide a process for the co-production of hydrocarbons

and alcohols. It is a further object of the invention to provide a process for making hydrocarbons from a hydrogen-lean gasification product obtained from the gasification of coal and similar substances, e.g., a product obtained from the underground gasification of coal. It is a still further object of the invention to increase the heating value of a gas initially obtained from gasification of coal, residua, oil shale, tar sands and similar carbonaceous substances by converting the carbon monoxide present in such a gas to methane, ethane and higher hydrocarbons as well as alcohols. It is a still further object of the invention to provide a commercially feasible process for co-production of substitute natural gas, LPG, gasoline, liquid hydrocarbons, and alcohols that can be mixed with gasoline in an integrated plant from a feed obtained by the gasification of coal, residua, oil shale, tar sands and similar carbonaceous materials through the use of known gasification technology. Other objects of the invention will be in part obvious and in part pointed out hereafter.

## Summary of the Invention

The present invention is based on the finding that by incorporating water vapor, usually in the form of steam, into a synthesis gas of the type disclosed in my patent number 4,139,551 and by using a catalyst of the type described below, it is possible to employ as a starting material a synthesis gas that is quite lean in respect to elemental hydrogen and still achieve a good yield and the other advantages attained when using the patented process. In accordance with the present process a product of the gasification of e.g. coal is used that contains

a stoichiometric deficiency of the hydrogen required for formation of hydrocarbons and the deficiency is made up by the hydrogen content of the added steam.

While we do not wish to be bound as to any particular mechanism of the reaction that occurs, it appears probable that the reaction proceeds in accordance with mechanism I or mechanism II given below or perhaps in accordance with both mechanisms.

Mechanism I

One possible mechanism involves a shift conversion followed by a synthesis as follows;

The reaction between carbon monoxide and water represented by the equation:

$$CO + H_2O \longrightarrow CO_2 + H_2$$

is known as the water gas shift conversion and produces elemental hydrogen which, as a component of the feed, can react with carbon monoxide to form hydrocarbons and alcohols as follows:

$$xCO + 2\,x\,H_2 \longrightarrow (-CH_2-)_x + x\,H_2O$$

Mechanism II

A second possible mechanism is illustrated by the following equation:

$$3CO + H_2O \longrightarrow (-CH_2-) + 2CO_2$$

The foregoing equation represents the so-called Kolbel-Engelhardt synthesis which is known to produce hydrocarbons. However, the catalysts previously proposed for this reaction have had activities so low as to make the process

unattractive commercially. The catalysts of the present invention, when prepared as described hereafter, exhibit substantially improved activity in this reaction.

The objects and advantages of the present invention are achieved in general by contacting steam and carbon monoxide in a hydrogen atmosphere and in synthesis proportions under synthesis conditions to form hydrocarbons and alcohols using a catalyst comprising hydrided titanium supported on a metal or inter-metallic compound capable of forming unstable hydrides, more particularly, hydrides that are unstable at a temperature within the range of atmospheric temperature to about 100°C. As pointed out more fully below, before being used in the synthesis process, the catalyst is desirably activated by alternate hydriding and dehydriding steps to cause a partial physical disintegration of the catalyst and thereby increase its surface area. The hydrogen atmosphere that is necessary for maintaining the catalytic activity may be provided by adding hydrogen to the feed mixture of steam and carbon monoxide. As pointed out hereafter, the amount of added hydrogen may vary over a wide range.

The catalysts of this invention for the first time permit the use of a steam/carbon monoxide feed to achieve commercially feasible hydrocarbon production rates. The cost of the feed stock is significantly reduced by substituting the relatively inexpensive steam for costly hydrogen. The catalysts of this invention make it possible to use hydrogen-lean synthesis gases produced, for example, by conventional gasification processes directly, i.e., without the need for an additional water gas shift step, in the syntheses of hydrocarbons and alcohols. Consequently, the cost of production of hydrocarbons and alcohols is substantially lowered. The catalysts of

the invention also for the first time permit the use of significantly lower operating temperatures and pressures in achieving commercially feasible production rates in the synthesis of alcohols. As the thermodynamics of the chemical system concerned permits higher equilibrium conversion at lower temperatures, the actual yields are greatly improved. Furthermore, again with respect to the alcohol synthesis, equipment requirements are much less stringent and less costly by reason of the lower operating pressures that can be employed at the process temperature selected. In addition, the use of the catalyst and the process of this invention greatly improves the economics of synthetic production of hydrocarbons and alcohols, which are valuable fuels and starting materials of many organic syntheses, by allowing the co-production of the same in an integral chemical complex which will produce the feed for the said synthesis through a more economical gasification process than heretofore possible,as well as employing a more economical process in the actual synthesis of hydrocarbons and alcohols.

The catalyst of this invention is free from many problems commonly encountered with prior art catalysts. For instance, there is little risk of carbonyl formation and also much less poisoning or deactivation of the catalyst through the smothering of adsorbing sites by the adsorption of impurities that normally exist in commercially available carbon monoxide. To the contrary, it has been found that the catalytic activity of the catalyst employed in the present invention increases with aging in an atmosphere containing hydrogen.

This enhancement of activity is the result of cracks and fissures formed in the catalyst particles, both microscopically (surface cracks) and macroscopically (breakage into smaller particles), with the attendant increase in the active surface area of the catalyst. Moreover, continuous cleaning of the catalyst surface from impurities by the hydrogen present in the reaction atmosphere prolongs the activity of the catalyst.

## General Description of Preferred Embodiment

As indicated above, the present catalysts comprise a titanium hydride and a support which may be either a metal or inter-metallic compound capable of forming unstable hydrides. As will be pointed out below, a wide variety of metals may be used as the supporting metal or in the supporting inter-metallic compound of the catalyst. However, the preferred support is an inter-metallic compound of iron and titanium.

The Fe/Ti alloy used in preparing the catalyst may be considered as comprising two phases, namely, (a) free titanium metal capable of being hydrided upon proper exposure to a hydrogen atmosphere and (b) an inter-metallic compound of iron and titanium in a 1:1 ratio that forms the support for the titanium metal, and after hydrogenation, for the titanium hydride. The support or carrier of phase (b) may be a commercially available alloy having the desired 1:1 ratio obtainable, for example, from the International Nickel Company. This alloy is further described in a book entitled "Constitution of Binary Alloys", First Supplement, by R. P. Elliott, published by McGraw Hill, New York, N. Y., 1965 and a paper of Reilly et al. referenced more fully below.

The carrier alloy is formed from relatively pure iron and titanium by a melting process requiring temperatures in the range of 1500° to 1900°C. Although the carrier can be made from commercial grade iron, the purity of electrolytic iron is preferred.

The supported catalyst comprising a separate titanium phase A may be made in the same way as the binary 1:1 alloy, since an alloyed mixture containing more than 1 mole of titanium for each mole of iron will produce a composition consisting of two phases, namely, the inter-metallic compound having a titanium to iron mole ratio of approximately 1:1 and pure titanium, the latter being converted to hydrided titanium in a hydriding step. The titanium hydride formed in this step is a non-stoichiometric hydride which contains slightly less hydrogen than the stoichiometric dihydride and may, for example, have the approximate formula $TiH_{1.98}$. This titanium hydride is a relatively stable compound which remains as such throughout the production process and is continually activated (decontaminated) as long as it is in contact with significant amounts of hydrogen in the synthesis process.

Although hydrided titanium appears to exhibit increased catalytic activity when alloyed with or otherwise supported on the iron-titanium bi-metallic compound, the catalytically active component is considered to be the hydrided titanium. For further information on the independent properties of each of these compounds, particular reference is made to the article entitled "Formation and Properties of Iron Titanium Hydride" by J. J. Reilly and R. H. Wiswall, Jr., of Brookhaven National Laboratory, published in Inorganic Chemistry, Vol. 13, No. 1, 1974, at pages 218-222, and to the book by W. M. Mueller, et al., entitled Metal Hydrides as published by Academic Press, New York, N. Y., 1968.

The preparation of the hydrided catalyst and its activation will now be described.

Activation Procedure

The titanium/iron alloy used in preparing the preferred catalyst of the invention is usually received from the manufacturer in granular form. The granular material as received is coated with an oxide layer and in this state neither the titanium phase (a) nor the inter-metallic phase (b) can form a hydride. Also the granules have other surface and internal impurities such as carbon and nitrogen compounds and adsorbed gases other than hydrogen and nitrogen. Initial cleaning of the particles and formation of the catalyst containing the titanium in hydrided form is desirably accomplished by charging the granular material as received into a sutiable reactor and subjecting it to hydrogen gas, preferably at temperatures in the range of 200°C. to 400°C. and at pressures in the range of 0 to 3000 p.s.i.g., preferably 150 to 200 p.s.i.g., for an extended period of time.

This initial exposure to hydrogen is followed by an outgassing step and then alternate pressurizing of the catalyst bed with hydrogen and outgassing so that phase (b) of the catalyst is successively hydrided and dehydrided. This process breaks up the catalyst granules into smaller particles and also produces multiple cracks in the surface of each individual granule, thereby greatly increasing the reactive surface area of the catalyst bed. The dehydriding step may be carried out at a temperature and hydrogen partial pressure that favors the decomposition of the hydride of phase (b) as determined by the theremodynamic equilibrium diagram of the inter-metallic compounds used. However, it is preferably carried out at or about 200°C. by drawing a slight vacuum of one or two inches of water on the reactor vessel containing the catalyst bed. Although the dehydriding may also be carried out by purging the reactor vessel with an inert gas such as helium, extreme care must be observed under such circumstances for the commercial grades of inert gases such as helium also contain oxygen at an impurity level which permanently poisons the catalyst by forming a stable oxide upon contact therewith at elevated temperatures.

The hydriding step may be carried out at a temperature and pressure that favors the formation of the hydride phase (b) as determined from the equilibrium conditions of the material used. Hydriding is preferably carried out at a temperature of 20° to 25°C. and at a pressure of the order of 1,000 psia.

Any commercially available grade of hydrogen gas may be used in the activation process. However, the use of relatively

high purity hydrogen permits the attainment of acceptable activation in a fewer number of cycles. Whether a less expensive grade of hydrogen or faster activation of the catalyst is desirable from the point of view of process economics, will depend upon the conditions at a given manufacturing plant.

If the hydrogen gas used in the activation process is less than 99.999% pure, then a high temperature hydrogen treatment step between the dehydriding and hydriding steps should be incorporated in the activation cycle. This added step is essentially the same as the initial hydrogen treatment and in this case removes the gases such as the impurities in the hydrogen gas used that become adsorbed on the catalyst particles during the hydriding step. Therefore, following the dehydriding step the reactor is filled with hydrogen gas at a temperature of about 200° to 400°C. and a pressure of approximately 150 to 200 psia preferably for four to six hours. Following this high temperature hydrogen treatment the hydrogen gas is again evacuated from the reactor by drawing a vacuum and the catalyst bed is cooled to 20° to 25°C. Upon reaching this temperature level, the reactor is pressurized with hydrogen gas at about 1000 psia and kept at these conditions until phase (b) is completely hydrided. The extent to which the hydriding reaction has proceeded may be followed by monitoring either temperature or pressure changes in the reactor and noting the time at which these parameters return to their initial values. Alternatively, the hydriding can be continued for a specific period of time, e.g., one-half hour.

After the hydriding reaction is completed the hydrogen is drawn off from the reactor by applying a vacuum until the pressure in the reactor is approximately one to two inches of water and concurrently the catalyst bed is heated to 200°C. in order to dehydride phase (b). The foregoing hydriding-dehydriding cycle is carried out until phase (b) of the catalyst is capable of being hydrided and dehydrided very rapidly. In the case of catalyst compositions that are to be prepared in powdered form, the hydriding and dehydriding cycles are continued until the desired particle size is attained.

Synthesis Process

The catalyst prepared as described above is charged to a suitable reactor and a gaseous feed stream comprised of hydrogen, carbon monoxide and water vapor is continuously passed over the catalyst bed in the reactor at a suitable temperature and pressure. The range of synthesis temperatures is governed by two counteracting factors. On the one hand, higher temperatures result in high reaction rates. On the other hand, the hydrogen concentration at the equilibrium of the water gas shift reaction is favored by lower temperatures. The practical temperature range appears to be about 150°C. to 350°C.

Higher pressures favor both the rate of production of the desired products and the equilibrium concentrations of these products. The reaction may be carried out at a pressure within the range 1 to 200 atmospheres. The preferred range appears to be 30 to 100 atmospheres.

As indicated above, the feed stream for the process may be obtained by the gasification of coal, residua, oil shale or tar sands by any of the well known gasification techniques. The technology used will vary according to the nature of the material to be gasified. The gasification product is then mixed with steam and in some cases hydrogen at selected ratios and fed to the reactor that contains the catalyst. A substantial proportion of the hydrogen is incorporated in the feed mixture to enhance the reaction of water vapor and carbon monoxide rather than to enter into the reaction itself. Therefore, hydrogen is separated from the product stream and continuously recycled. However, the hydrogen may be gradually consumed to a relatively small extent by reaction with carbon monoxide and/or carbon dioxide. In that case a replenishment of the hydrogen so consumed is necessary and this may be accomplished for instance either by increasing the steam/carbon monoxide ratio or by adding hydrogen to the system.

In the production of the synthesis gas, the water/gas shift step which is ordinarily carried out subsequent to the gasification process to adjust the hydrogen/carbon monoxide ratio or to generate hydrogen may be omitted when the catalyst and process of the present invention is used. Alternatively, a small proportion of the gasification product may be subjected to the shift reaction. Since hydrogen is effectively produced during the synthesis process, significant cross-reductions are achieved.

The amount of added hydrogen will depend to some extent on the hydrogen content of the synthesis gas. As indicated above, the proportion of hydrogen in the feed mixture can vary

over a wide range, i.e., from a molar ratio of hydrogen to steam/carbon monoxide mixture of about 1:9 to 99:1. The minimum amount of hydrogen is about 10 volume %.

The ratio of CO to steam does not appear to be particularly critical and may be varied over a relatively wide range depending on the product or products to be produced. In most cases this ratio should desirably be greater than 1.5:1.

Other Embodiments of the Invention

Although a single embodiment of the present invention has been described, other useful embodiments and variations of the catalyst composition, the mode of preparation and treatment of the catalyst and synthesis steps will be apparent to those skilled in the art. As indicated above, the catalyst may broadly comprise a hydrided metal and a support comprising a metal or inter-metallic compound characterized by the fact that it is capable of forming an unstable hydride. The hydrided metal may be hydrided titanium as described in detail above, or alternatively hydrided zirconium. Of the elemental metals that may be used as a support, vanadium, zirconium, and niobium may be mentioned. A considerable number of inter-metallic compounds may be used, including compounds of titanium with metal selected from the group consisting of iron, nickel, cobalt, chromium, molybdenum and manganese. Amoung the specific inter-metallic compounds that may be mentioned are those of the following formulas: FeTi, $NiTi_2$, TiCo, $TiCr_2$, $ZrNi_2$, $ZrMo_2$, $ZrFe_2$, $ZrCo_2$, $ZrMn_2$, $TiFe_{0.8}Mn_{0.2}$, $TiFe_{0.9}V_{0.1}$, $TiFe_{0.8}Cr_{0.2}$, $TiFe_{0.8}Mo_{0.2}$, $TiCo_{0.8}Mn_{0.2}$, $TiCo_{0.9}Fe_{0.1}$, $TiCo_{0.8}V_{0.2}$, $TiCo_{0.9}Ni_{0.1}$, $TiCo_{0.8}V_{0.2}$, $TiCo_{0.9}Ni_{0.1}$, $TiCr_{1.8}Fe_{0.2}$, $TiCr_{1.8}V_{0.2}$, $TiCr_{1.7}Mo_{0.3}$, $VTi_2$, $NiTi_3$, $CoTi_2$, and CrTi.

Other examples of inter-metallic compounds capable of forming hydrides that are unstable at temperatures up to 100°C. can be found in the literature.

Other variations within the scope of the invention involve combining titanium dihydride with catalytically active metals for this reaction such as ruthenium and palladium either in the form of mixtures of multi-component (e.g. ternary, quaternary or higher) alloys, or supporting this catalyst upon other inter-metallic compounds or on an inert carrier material or other substrates.

By way of further example, activation of the catalyst can be achieved, although at a slower rate, by exposing the catalyst to hydrogen in the feed stream of the synthesis process itself, particularly, if the product stream is recycled until a desired product concentration is achieved. It is therefore to be understood that the foregoing specification is not intended to be limited to the preferred embodiment of the invention described and that other embodiments are contemplated within the scope of the appended claims.

1. A method of making hydrocarbons and alcohols which comprises contacting a gaseous mixture of steam, hydrogen and carbon monoxide in synthesis proportions at synthesis conditions with a catalyst comprising hydrided titanium supported on a metal or inter-metallic compound capable of forming an unstable hydride.

2. A method of making hydrocarbons and alcohols which comprises contacting a gaseous mixture of steam, hydrogen and carbon monoxide in synthesis proportions at synthesis conditions with a catalyst comprising hydrided titanium supported on a metal or inter-metallic compound capable of forming hydrides that are unstable at atmospheric temperature.

3. A method of making hydrocarbons and alcohols which comprises contacting a gaseous mixture of steam, hydrogen and carbon monoxide in synthesis proportions at synthesis conditions with a catalyst comprising hydrided titanium supported on an inter-metallic compound capable of forming an unstable hydride.

4. A method according to claim 3 wherein said catalyst is made by preparing a composition comprising free titanium and said inter-metallic compound and exposing said composition to hydrogen at an elevated temperature to convert free titanium thereof to hydrided titanium.

5. A method according to claim 4 wherein said catalyst is activated by alternately hydriding it at ambient temperature and dehydriding it at an elevated temperature to increase the surface area of said catalyst.

6. A method according to claim 5 wherein said catalyst is hydrided at a pressure above atmospheric and dehydrided at a pressure below atmospheric..

7. A method according to claim 3 wherein the volumetric ratio of hydrogen to steam and carbon monoxide in said gaseous mixture is from about 1:9 to 99:1.

8. A method according to claim 3 wherein the inter-metallic compound is a compound of titanium or zirconium with a metal selected from the group consisting of iron, nickel, cobalt, chromium, molybdenum and manganese.

9. A method according to claim 8 wherein the inter-metallic compound is a compound of titanium and iron.

10. A method according to claim 9 wherein the molar ratio of titanium to iron in the inter-metallic compound is about 1:1.

11. A method of making hydrocarbons and alcohols which comprises contacting a gaseous mixture of steam, hydrogen and carbon monoxide in synthesis proportions at synthesis conditions with a catalyst prepared by alloying titanium with a second metal capable of forming an inter-metallic compound with titanium under alloying conditions such as to produce an inter-metallic compound of titanium and said second metal as well as free titanium in an amount effective when hydrided to catalyze the conversion of steam, hydrogen and carbon monoxide to hydrocarbons and alcohols and then exposing the alloy of said second metal and titanium to hydrogen under hydriding conditions such as to hydride at least a portion of said free titanium.

12. A method according to claim 11 in which said second metal is selected from iron, nickel, cobalt, chromium, molybdenum and manganese.

13. A method according to claim 11 wherein said hydriding conditions are such as to remove oxides from the surface of said alloy.

14. A method according to claim 11 wherein said inter-metallic compound is a compound of titanium and iron.

0035328

European Patent Office

# EUROPEAN SEARCH REPORT

Application number
EP 81 30 0527.9

## DOCUMENTS CONSIDERED TO BE RELEVANT

| Category | Citation of document with indication, where appropriate, of relevant passages | Relevant to claim |
|---|---|---|
| A | FR - A1 - 2 266 676 (THE REGENTS OF THE UNIVERSITY OF CALIFORNIA)<br>* claims 1, 5 * | 1 |
| A | FR - A1 - 2 380 239 (RAFEL INDUSTRIAL GROUP)<br>* claims 1, 8 * | 1 |
| D | & US - A - 4 139 551 | |

CLASSIFICATION OF THE APPLICATION (Int. Cl.³)

C 07 C 1/22
C 07 C 29/15
B 01 J 31/38

TECHNICAL FIELDS SEARCHED (Int. Cl.³)

B 01 J 31/02
B 01 J 31/26
B 01 J 31/38
C 07 C 1/20
C 07 C 1/22
C 07 C 29/15

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| Berlin | 03-06-1981 | KNAACK |